# EUROPEAN PATENT APPLICATION

(11) **EP 0 797 955 A1**
(43) Date of publication of application: **01.10.1997**
(21) Application number: 97301545.6
(22) Date of filing: 07.03.1997
(51) Int. Cl.: A61B 17/17

(54) **Surgical instrument for anterior cruciate ligament reconstruction**

(30) Priority: 28.03.1996 GB 9606525
(71) Applicant: UNITED SURGICAL SERVICES LTD., Worcester WR1 3DG (GB)
(72) Inventor: Strover, Angus Everett, Worcester WR1 3DG (GB)
(74) Representative: Jackson, Derek Charles

(57) **Abstract**

An ACL drill jig is described for locating a tunnel to be drilled through a femur (27) for fixation of a ligament graft between the femur and a tibia (29). The drill guide includes a member (1) having first and second ends (3, 5), the member being non-linear intermediate its ends. A cylindrical bore is formed at the first end (3) of the member (1) for receiving a guide tube (9), the cylindrical bore having an axis. A channel portion (15) is provided at the second end (5), the channel portion defining a channel (19) substantially coaxial with the axis of the cylindrical bore. A tongue portion (17) is provided at that end of the channel portion (15) remote from the cylindrical bore for engaging a notch (25) in a femur (27), the tongue portion (17) being offset from the channel (19) of the channel portion for aligning the axis of the cylindrical bore and of the channel at a predetermined offset location in the notch.

## Description

This invention relates to surgical instruments for use in the procedure for reconstruction of the anterior cruciate ligament, hereinafter referred to as ACL. In particular, the present invention relates to an ACL drill jig for locating a tunnel to be drilled through a femur for fixation of a ligament graft between the femur and a tibia.

The surgical procedure for reconstruction of the ACL entails replacement of the damaged ligament by either a biological graft (autograft or allograft) or by an artificial ligament. The replacement is positioned through bone tunnels to emerge from the anatomical attachment sites (insertions) of the ACL on the tibia and femur within the intercondylar notch of the knee joint.

When this procedure is performed by arthroscopy (endoscopy of the joint), the locations of emergence of bone tunnels within the knee joint are aimed at being placed precisely in the insertions of the broken or absent ACL. To this end these anatomical locations are usually made with the assistance of jigs, by reference to the visible anatomical structures in the knee joint. Jigs presently available are, however, potentially inaccurate because, due to the nature of the two-dimensional image used in arthroscopy, the third dimensional distance, or depth, can be deceptive. Finally the presence of bleeding and of soft tissue remnants of the broken ligament often makes visualisation of the anatomical landmarks difficult and malpositioning of the tibial and femoral bone tunnels remains the most common surgical error in reconstructing the ACL.

The object of the present invention is to provide an ACL drill jig able to locate a bone tunnel within the femur at the intra-articular attachment sites of the ACL by reference to the postero-superior boundary of the intercondylar notch of the femur.

According to the present invention there is provided an ACL drill jig for locating a tunnel to be drilled through a femur for fixation of a ligament graft between the femur and a tibia, the drill guide comprising:
a member having first and second ends, the member being non-linear intermediate the ends thereof;
a cylindrical bore formed at the first end of the member for receiving a guide tube, the cylindrical bore having an axis;
a channel portion provided at the second end, the channel portion defining a channel substantially coaxial with the axis of the cylindrical bore; and
a tongue portion provided at that end of the channel portion remote from the cylindrical bore for engaging a notch in a femur, the tongue portion being offset from the channel of the channel portion for aligning the axis of the cylindrical bore and of the channel at a predetermined offset location in the notch.

The member may be curved intermediate the ends thereof, such as substantially arcuate.

The cross-sectional area of the member may decrease towards the second end.

The cylindrical bore may be provided with an axially extending slot.

The channel may be substantially semi-cylindrical.

Releasable locking means may be provided at the first end of the member for releasably locking the guide tube in any one of a range of selected positions.

The tongue portion may be generally parallel to the channel.

A tibial fixation pin may be provided intermediate the ends of the member. The tibial fixation pin may be directed towards that end of the channel proximal to the cylindrical bore. The tibial fixation pin may be positioned at an angle of about 27.5 degrees to the axis of the cylindrical bore and the channel.

For a better understanding of the present invention and to show more clearly how it may be carried into effect reference will now be made, by way of example, to the accompanying drawings in which:
Figure 1 is a side elevational view of one embodiment of an ACL drill jig according to the present invention;
Figure 2 is an end elevational view of part of the jig shown in Figure 1; and
Figure 3 is a view illustrating the jig of Figures 1 and 2 in use.

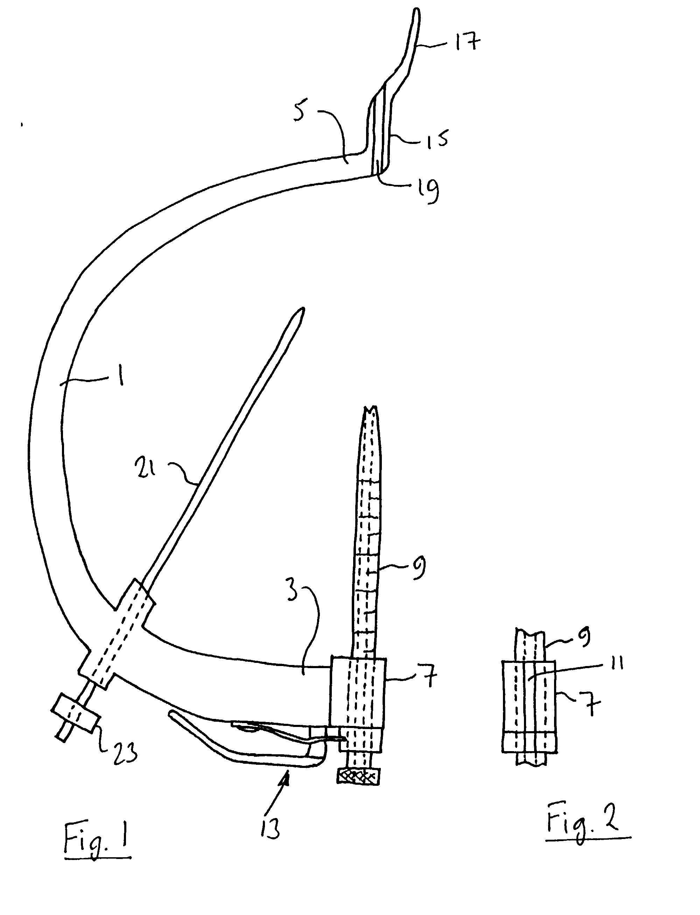

The ACL drill jig shown in Figures 1 to 3 comprises a generally arcuate, curved member 1 which may be made of generally rectangular cross-section material, the width of the member 1 decreasing from one end 3 thereof towards the other 5 as illustrated in order that the end 5 can be inserted through an arthroscopic portal in a patient's knee. The end 5 will in practice be the lower end of the jig and the end 3 will in practice be the upper end thereof.

The lower end 5 of the member 1 is formed with a head portion 7 having a cylindrical bore formed therethrough for receiving a cannulated guide tube 9, the guide tube 9 being slidable in the bore so as to permit insertion and removal from the bore in an axial direction. The cylindrical bore is additionally formed with an axially slot 11 extending from the internal surface of the bore to the external surface of the head portion 7, the slot being dimensioned such that a guide wire can be passed into and, more particularly, removed from the cylindrical bore in the absence of the guide tube 9. Thus, for example, the slot may have a width of about 2 to 3 mm.

Graduations are etched on the guide tube to indicate the depth of bone to be traversed from the outer tibial cortex to the entrance of the intercondylar notch. The end 10 of the guide tube is tapered so as to form a point or blade which can be driven into the tibia to assist in securing the jig and guide tube in the required position.

The lower end 5 of the jig may be provided with a releasable locking mechanism 13 for releasably locking the guide tube 9 in any one of a range of selected positions.

The upper end 3 of the member 1 is provided with a channel portion 15 and a tongue portion 17. The channel portion 15 is provided with a generally semi-cylindrical channel 19 coaxial with the cylindrical bore of the head portion 7 and with the bore of the guide tube 9. The tongue portion 17 extends generally parallel to the channel portion, but is offset laterally therefrom by about 7 mm on that side of the channel portion 15 opposite the curved member 1. However, alternative offsets can be employed if desired.

Intermediate the upper end 3 and the lower end 5 of the curved member 1 there is optionally provided a cylindrical bore for receiving a tibial fixation pin 21. The tibial fixation pin is preferably directed towards that end of the channel 19 proximal the head portion 7 and is positioned at an angle of about 27.5 degrees to the axis passing through the cylindrical bore and the channel 19. The tibial fixation pin 21 may be provided with means 23 for limiting the movement thereof towards the semi-cylindrical channel 19. The tibial fixation pin may have a screw-threaded tip to enable it to be screwed into the tibia.

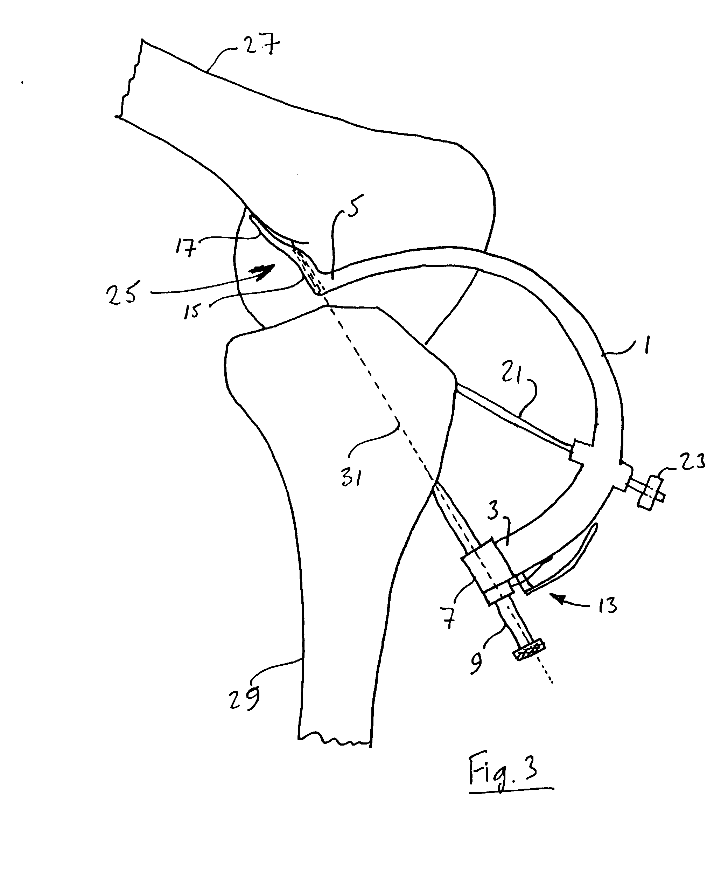

In use of the jig as illustrated in Figure 3 the tongue portion 17 and channel portion 15 at the upper end 3 of the member 1 are inserted through an arthroscopic portal of about 5 mm positioned close to the medial border of the patellar tendon about 10 mm proximal to the antero-medial edge of the tibial plateau.

Guided by arthroscopic means, the tongue portion 17 and channel portion 15 are guided into the intercondylar notch 25 of the femur 27 (suitably cleaned of remnants of the broken ligament and unwanted osteophytes).

The proximal end of the channel portion 15 is placed between the medial and lateral tibial spines and the tongue portion 17 is hooked behind the posterior edge of the intercondylar notch 25.

The guide tube 9 is then passed through the bore in the head portion 7 and into a bone tunnel formed in the tibia 29 in order to secure the jig in position. Additional securement may be by way of the tibial fixation pin 21 which may be drilled into the tibia 29 by way of a small stab wound in the shin.

A guide wire 31 (illustrated by a dashed line in Figure 3) is then passed through the guide tube 9 and tibial bone tunnel and along the semi-cylindrical channel 19 until it contacts the femur in a position predetermined by the offset of the tongue portion 17. The guide wire 31 can then be drilled into the femur in order to provide an accurate indication of the required location of the femoral bone tunnel.

Once the guide wire 31 has been positioned, the guide tube 9 is withdrawn, together with any tibial fixation pin 21, and the jig can be withdrawn from the patient by passing the guide wire through the slot 11 and withdrawing the channel portion 15 and tongue portion 17 through the arthroscopic portal. The femoral tunnel can then be drilled either with a cannulated drill or bone-corer (not shown) passing over the guide wire 31, or by additionally withdrawing the guide wire 31 and passing a separate drill through the tibial tunnel and into the site previously identified by the guide wire.

## Claims

1. An ACL drill jig for locating a tunnel to be drilled through a femur (27) for fixation of a ligament graft between the femur and a tibia (29), characterised in that the drill guide comprises:
a member (1) having first and second ends (3, 5), the member being non-linear intermediate the ends thereof;
a cylindrical bore formed at the first end (3) of the member (1) for receiving a guide tube (9), the cylindrical bore having an axis;
a channel portion (15) provided at the second end (5), the channel portion defining a channel (19) substantially coaxial with the axis of the cylindrical bore; and
a tongue portion (17) provided at that end of the channel portion (15) remote from the cylindrical bore for engaging a notch (25) in a femur (27), the tongue portion (17) being offset from the channel (19) of the channel portion for aligning the axis of the cylindrical bore and of the channel at a predetermined offset location in the notch.

2. An ACL drill jig as claimed in claim 1, characterised in that the member (1) is curved intermediate the ends (3, 5) thereof, such as substantially arcuate.

3. An ACL drill jig as claimed in any preceding claim, characterised in that the cross-sectional area of the member (1) decreases towards the second end (5).

4. An ACL drill jig as claimed in any preceding claim, characterised in that the cylindrical bore is provided with an axially extending slot (11).

5. An ACL drill jig as claimed in any preceding claim, characterised in that the channel (19) is substantially semi-cylindrical.

6. An ACL drill jig as claimed in any preceding claim, characterised in that releasable locking means (13) is provided at the first end (3) of the member (1) for releasably locking the guide tube (9) in any one of a range of selected positions.

7. An ACL drill jig as claimed in any preceding claim, characterised in that the tongue portion (17) is generally parallel to the channel (19).

8. An ACL drill jig as claimed in any preceding claim, characterised in that a tibial fixation pin (21) is provided intermediate the ends of the member (1).

9. An ACL drill jig as claimed in claim 8, characterised in that the tibial fixation pin (21) is directed towards that end of the channel (19) proximal to the cylindrical bore.

10. An ACL drill jig as claimed in claim 8 or 9, characterised in that the tibial fixation pin (21) is positioned at an angle of about 27.5 degrees to the axis of the cylindrical bore and the channel (19).
